# EUROPÄISCHE PATENTSCHRIFT

(11) **EP 1 620 394 B1**
(45) Veröffentlichungstag und Bekanntmachung des Hinweises auf die Patenterteilung: **13.08.2008**
(21) Anmeldenummer: 04726856.0
(22) Anmeldetag: 10.04.2004
(51) Int. Cl.: C07C 277/08, C07C 279/10, C07C 315/06, C07C 317/44

(54) **VERFAHREN ZUR KRISTALLISATION VON GUANIDINIUM-SALZEN**
METHOD FOR CRYSTALLIZING GUANIDINIUM SALTS
PROCEDE POUR CRISTALLISER DES SELS DE GUANIDINIUM

(30) Priorität: 06.05.2003 DE 10320016
(43) Veröffentlichungstag der Anmeldung: 01.02.2006
(73) Patentinhaber: Merck Patent GmbH, 64293 Darmstadt (DE)
(72) Erfinder: KIRSCHBAUM, Michael, 64331 Weiterstadt (DE); BARTMANN, Ekkehard, 64390 Erzhausen (DE); BENSINGER, Dieter, 64291 Darmstadt (DE); HAAS, Alexander, 64297 Darmstadt (DE); LIPPERT, Ricky, 64395 Brensbach (DE)
(86) Internationale Anmeldenummer: PCT/EP2004/003838
(87) Internationale Veröffentlichungsnummer: WO 2004/099125

(56) Entgegenhaltungen:
- EP-A- 0 758 644
- US-A- 3 975 533
- US-A- 5 840 761

## Beschreibung

Die Erfindung betrifft ein Verfahren zur Kristallisation der Verbindungen der Formel I oder deren Säureadditions-Salze, 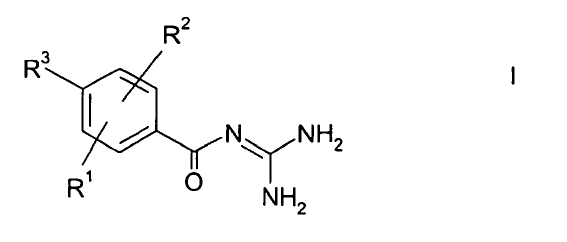 worin
- R¹: R⁴SO₂- oder A,
- R² und R³: unabhängig voneinander H, Hal, Alkyl mit 1 bis 12 C-Atomen, R⁴SO₂-, Ar oder Het,
- R⁴: Aryl oder Alkyl mit 1 bis 12 C-Atomen,
- Het: einen unsubstituierten oder einfach oder mehrfach durch A, COAr, COHet und/oder Hal substituierten, gesättigten, ungesättigten oder aromatischen, mono- oder bicyclischen, heterocyclischen oder linearen oder verzweigten, ein oder mehrere Heteroatome enthaltenden organischen Rest,
- Ar: einen unsubstituierten oder einfach oder mehrfach durch A und/oder Hal, OH, OA, COOH, COOA, CONH₂, CONA₂, CONHA, CN, NO₂, NH₂, NHA, NA₂, NHCOA, CF₃ oder SO₂A substituierten Phenylrest,
- A: geradkettiges oder verzweigtes Alkyl oder Hydroxyalkyl mit 1 bis 10 C-Atomen, Alkenyl oder Alkoxyalkyl mit 2 bis 10 C-Atomen,
und
- Hal: F, Cl, Br, I
dadurch gekennzeichnet, dass man die jeweiligen Verbindungen der Formel I oder deren Mischungen mit Verunreinigungen bei gegebener Temperatur in Wasser, das mit mindestens einem mit Wasser nicht mischbaren Lösungsmittel annähernd gesättigt ist und gegebenenfalls ein oder mehrere mit Wasser mischbare Lösungsmittel enthält, löst und bei niedrigerer Temperatur die Verbindungen der Formel I kristallisieren lässt, wobei als mit Wasser nicht mischbare Lösungsmittel Alkansäurealkylester oder aromatische Lösungsmittel verwendet werden.

Die Verbindungen der Formel I können ein oder mehrere chirale Zentren aufweisen. Sie können dementsprechend in verschiedenen enantiomeren Formen auftreten und in racemischer oder in optisch aktiver Form vorliegen. Gegenstand der Erfindung sind deshalb auch die optisch aktiven Formen (Stereoisomeren), die Enantiomeren, die Racemate, die Diastereomeren sowie Hydrate und Solvate dieser Verbindungen. Erfindungsgemäß sind auch die tautomeren Formen der Verbindungen der Formel I.

Sulfonyl-benzoylguanidine sind bekannt und beispielsweise beschrieben in EP 0 758 644 A1. Bei diesen Substanzen handelt es sich um Inhibitoren des zellulären Na⁺/H⁺-Antiproters, d.h. um Wirkstoffe, die den Na⁺/H⁺-Austauschmechanismus der Zellen hemmen (Düsing et al., Med. Klin. 1992, 87, 367-384) und die somit gute Antiarrythmika darstellen, die sich insbesondere zur Behandlung von Arrythmien eignen, die als Folge von Sauerstoffmangel auftreten.

Diese Substanzen zeigen eine gute kardioprotektive Wirkung und eignen sich daher besonders zur Behandlung des akuten Myokardinfarkts, der Infarkt-Prophylaxe, Post-Infarktbehandlung, chronischer Herzinsuffizienz und zur Behandlung von Angina pectoris. Ferner wirken sie allen pathologischen hypoxischen und ischämischen Schädigungen entgegen, so daß die dadurch primär oder sekundär verursachten Krankheiten behandelt werden können. Diese Wirkstoffe sind ebenfalls für präventive Anwendungen gut geeignet.

Aufgrund der protektiven Wirkung dieser Substanzen bei pathologischen hypoxischen oder ischämischen Situationen resultieren daraus weitere Anwendungsmöglichkeiten bei chrirurgischen Eingriffen zum Schutz zeitweilig minderversorgter Organe, bei Organtransplantationen zum Schutz der entnommenen Organe, bei angioplastischen Gefäß- oder Herzeingriffen, bei Ischämien des Nervensystems, bei der Therapie von Schockzuständen und zur Verhinderung der essentiellen Hypertonie.

Ferner können diese Verbindungen auch als Therapeutika bei durch Zellproliferation bedingten Erkrankungen wie Arteriosklerose, Diabetes und diabetischen Spätkomplikationen, Tumorerkrankungen, fibrotischen Erkrankungen, insbesondere von Lunge, Leber und Nieren sowie Organhypertrophien und -hyperplasien, eingesetzt werden. Darüber hinaus eignen sich die Verbindungen zu diagnostischen Anwendung zur Erkennung von Krankheiten, die von einer gesteigerten Aktivität des Na⁺/H⁺-Antiporters z. B. in Erythrozyten, Thrombozyten oder Leukozyten begleitet werden.

Die Verbindungen können daher Arzneimittelwirkstoffe in der Human- und Veterinärmedizin verwendet werden. Ferner können sie als Zwischenprodukte zur Herstellung weiterer Arzneimittelwirkstoffe Verwendung finden.

Verbindungen der Formel I sind beispielsweise herstellbar nach EP 0 758 644. Üblicherweise fallen die Wirkstoffe bei diesen Verfahren mit einem Gehalt von 95 bis 99 HPLC-Flächenprozent an, was nicht den Anforderungen an pharmazeutische Wirkstoffe entspricht. Eine zusätzliche Aufreinigungsoperation ist erforderlich.

Eine Umkristallisation der Produkte aus Wasser oder den üblichen organischen Lösungsmittel ist jedoch praktisch nicht möglich. Neben der nur geringen Löslichkeit der Produkte (auch in der Hitze), die schlechte Kristallisationausbeuten bedingen, erhält man Kristalle unzureichender Reinheit. Auch eine mehrfache Kristallisation aus Wasser oder den üblichen organischen Lösungsmitteln führt nicht zu Material ausreichender Reinheit.

Es ist möglich, die Aufreinigung des Rohproduktes durch Lösen in sehr viel Wasser und anschließendes Einengen der wässrigen Lösung im Vakuum auf einen Bruchteil des ursprünglichen Volumens zu erreichen, wobei das Produkt auskristallisiert. Nachteil dieses Verfahrens sind die sehr lange Prozessdauer (das Einengen der wässrigen Lösungen benötigt bei Großansätzen mehrere Tage) und die dadurch bedingten Produktverluste durch Hydrolyse.

Aufgabe der vorliegenden Erfindung war es daher, ein verbessertes Kristallisationsverfahren für die Verbindungen der Formel I und deren Säureadditions-Salze bereitzustellen, das in großtechnischen Maßstäben anwendbar ist.

Diese Aufgabe wurde gelöst durch das erfindungsgemäße Verfahren zur Kristallistion der Verbindungen der Formel I, das dadurch gekennzeichnet ist, dass man die jeweiligen Verbindungen der Formel I oder deren Mischungen mit Verunreinigungen bei gegebener Temperatur in Wasser, das mit mindestens einem mit Wasser nicht mischbaren Lösungsmittel annähernd gesättigt ist und gegebenenfalls ein oder mehrere mit Wasser mischbare Lösungsmittel enthält, löst und bei niedrigerer Temperatur die Verbindungen der Formel 1 kristallisieren lässt, wobei als mit Wasser nicht mischbare Lösungsmittel Alkansäurealkylester oder aromatische Lösungsmittel verwendet werden.

In den Verbindungen der Formeln 1 weisen die Reste folgende bevorzugte Bedeutungen auf:
R¹ bedeutet vorzugsweise R⁴SO₂- oder A.
R² befindet sich in den Verbindungen der Formel I bevorzugt in ortho-Position zum Guanidin-Rest und bedeutet vorzugsweise H oder Alkyl mit 1 bis 7 C-Atomen, insbesondere H oder Methyl.
R³ bedeutet vorzugsweise H, Alkyl mit 1 bis 7 C-Atomen, R⁴SO²- oder Het, insbesondere R⁴SO₂- oder Het.
R⁴ bedeutet vorzugsweise Phenyl, m-, o- oder p-Tolyl oder Methyl, Ethyl, iso-Propyl, n-Butyl oder n-Pentyl. Besonders bevorzugt sind Methyl oder Ethyl, insbesondere Methyl.
Ar bedeutet vorzugsweise Phenyl, m- o- oder p-Methyl oder
   Methoxyphenyl.
Het bedeutet vorzugsweise 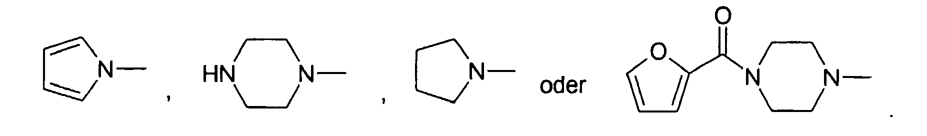
Hal bedeutet vorzugsweise Cl oder Br, insbesondere F oder Cl. Das erfindungsgemäße Verfahren eignet sich insbesondere zur Kristallisation von Verbindungen der Formel la bis le oder deren Säureadditionssalze:

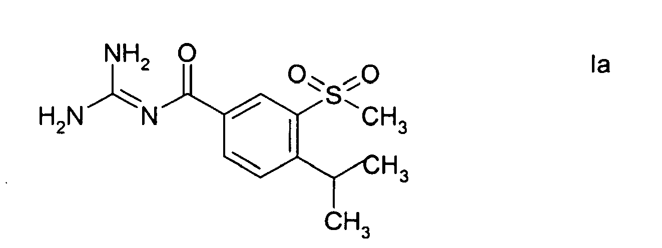

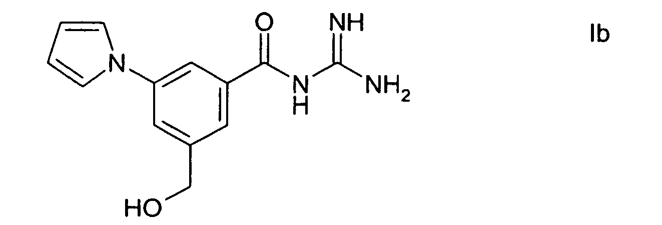

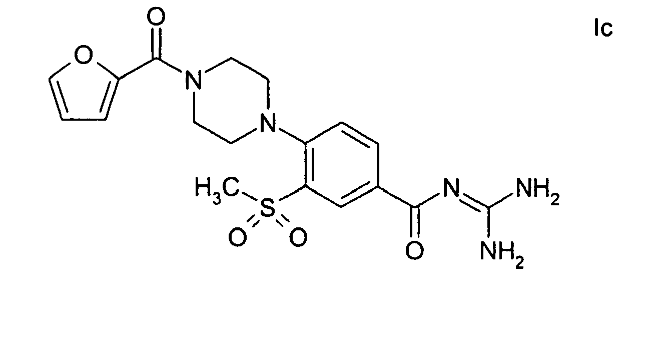

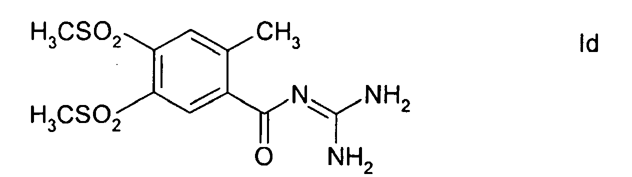

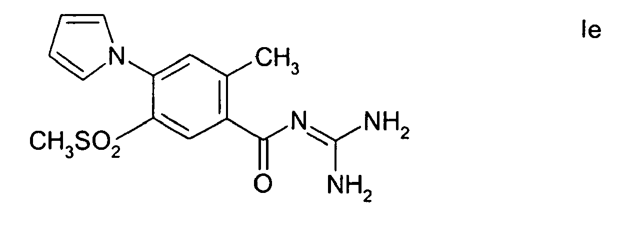

Die Reaktionsdurchführung des erfindungsgemäßen Verfahrens ist einfach, wobei man die Verbindungen der Formel I umkristallisieren kann wenn man als Solvens Wasser verwendet, das mit einem mit Wasser nicht mischbaren Lösungsmittel annähernd gesättigt ist. Dies ist völlig unerwartet, weil die Löslichkeit der Verbindungen der Formel I in den reinen Lösungsmitteln, d. h. in Wasser oder in einem oder mehrern mit Wasser nicht mischbaren Lösungsmittel, nicht ausreicht, um ein effektives Umkristallisieren zu ermöglichen. Mit dem erfindungsgemäßen Solvens-System kann jedoch eine hervorragende Raum-Zeitausbeute bei gleichzeitig sehr guter Reinheit (> 99,7 HPLC-Flächenprozent) erzielt werden, auch bei Großansätzen.

Zu praktischen Ausführungen ist es nicht notwendig, das Sättigungsmischungsverhältnis der beiden Solventen genau einzustellen. Bevorzugt verwendet man einen Überschuß an nicht mit Wasser mischbarem Lösungsmittel. Nach dem Lösen des Produktes in dem Solvensgemisch bei erhöhten Temperaturen trennt man vorzugsweise das überschüssige, nicht mit Wasser mischbare Lösungsmittel ab und lässt die Lösung zum Auskristallisieren abkühlen. Da die unvollständige Abtrennung des überschüssigen, nicht mit Wasser mischbaren Lösungsmittels sich nicht negativ auf die Kristallisation auswirkt, kann auch direkt aus dem Zweiphasengemisch kristallisiert werden.

Als nicht mit Wasser mischbare Lösungsmittel kommen für das erfindungsgemäße Verfahren generell alle bekannten, in erster Näherung nicht wassermischbaren Solventien in Frage. Vorzugsweise sind dies längerkettige nicht mit Wasser mischbare Ketone wie z.B. Methylethylketon oder Alkansäurealkylester, wie z.B. Ethylacetat, Isopropylacetat, Methylacetat oder Ethylpropionat. Weiterhin sind bevorzugt aromatische Lösungsmittel oder höhere Alkohole wie z. B. Butanol verwendbar. Insbesondere bevorzugt sind Toluol oder Xylol. Nach Vergleich aller relevanten Parameter (Ausbeute, Reinheit, Preis, Umweltverträglichkeit etc.) sind Alkansäurealkylester, insbesondere Ethylacetat jedoch anderen Solventen vorzuziehen.

Das erfindungsgemäße Verfahren lässt sich in der praktischen Anwendung noch weiter verfeinern, wenn man zu Mehrkomponenten-Gemischen übergeht. Bevorzugt ist ein Gemisch aus Wasser, einem nicht mit Wasser mischbaren Lösungsmittel und einem Alkohol, insbesondere eine Mischung aus Wasser, Ethanol und Essigester. Bevorzugt wird in Konzentrationsbereichen gearbeitet, in denen der zum Lösungsmittelgemisch zugesetzte Alkohol noch keine völlige Mischbarkeit von Wasser und mit Wasser nicht mischbaren Lösungmittel vermittelt. Als Alkohole sind insbesondere vorzugsweise Ethanol, Methanol oder n- oder iso- Propanol verwendbar.

Statt des Alkohols können auch Ketone und Nitrile verwendet werden. Bevorzugt sind wasserlösliche Ketone, insbesondere Aceton.

Vorzugsweise werden die Verbindungen der Formel I bei erhöhten Temperaturen, bevorzugt bei 30-180°C, insbesondere bei 60-100°C und ganz besonders bevorzugt bei 60-70°C, in dem jeweiligen Solvensgemisch, gelöst und bei niedrigeren Temperaturen, vorzugsweise bei Raumtemperatur zur Kristallisation gebracht.

Die Dauer der Umsetzung der Kristallisation hängt von den gewählten Reaktionsbedingungen ab. In der Regel beträgt die Kristallisationsdauer 0.5 Stunden bis 2 Tage, vorzugsweise 1 bis 15 Stunden.

In einer bevorzugten Ausführungsform des erfindungsgemäßen Kristallisationsverfahrens wird vor oder während der Kristallisation der pH-Wert mit Hilfe einer geeigneten Säure (z.B. bei Hydrochloriden mit HCl, bei Methansulfonat mit Methansulfonsäuren) auf 1 bis 3,5, insbesondere 1 bis 2 eingestellt.
Als Säuren, die vor oder während der Kristallisation zugesetzt werden, kommen weiterhin solche in Betracht, die mit den Verbindungen der Formel I physiologisch unbedenkliche und verträgliche Salze bilden.

Bevorzugt können hierfür anorganische Säuren verwendet werden, z.B. Schwefelsäure, Salpetersäure, Halogenwasserstoffsäuren, wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphtalinmono- und disulfonsäuren, Laurylschwefelsäure. Insbesondere ist Chlorwasserstoffsäure oder Methansulfonsäure bevorzugt.

Die Mengen der Lösungsmittelgemische für die erfindungsgemäße Kristallisation ist nicht kritisch, vorzugsweise können 10 g bis 500 g Lösungsmittelgemische je g der zu lösenden Verbindungen der Formel I verwendet werden.

Auch ohne weitere Ausführungsformen wird davon ausgegangen, daß ein Fachmann die obige Beschreibung im weitesten Umfang nutzen kann. Die bevorzugten Ausführungsformen sind deswegen lediglich als beschreibende, keineswegs als in irgendeiner Weise limitierende Offenbarung aufzufassen.

Die folgenden Beispiele sollen die Erfindung erläutern, ohne sie zu begrenzen. Sofern nicht anderes angegeben ist, bedeuten Prozentangaben Gewichtsprozent. Alle Temperaturen sind in Grad Celsius angegeben.

### Beispiel 1:

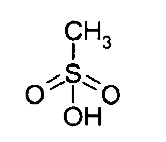

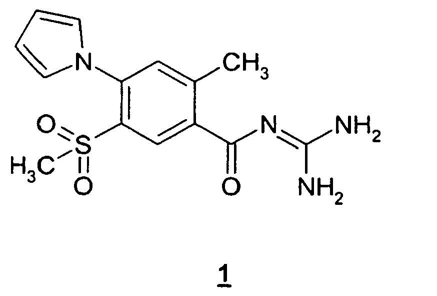

Zu 550 ml Wasser werden unter Rühren bei etwa 73°C 30,00 g der aufzureinigenden Verbindung 1 und 300 ml Ethylacetat gegeben.
Die Mischung wird bei 75°C für 40 Min. gerührt, wobei zwei klare Phasen entstehen. Die wässrige Phase wird abgetrennt und über einen Wasserdampf beheizten 21-Seitzfilter (Filter K 900) gegeben.
Man lässt die wässrige Phase über Nacht unter Rühren abkühlen und rührt für weitere 3 Stunden unter Eiskühlung. Die entstandenen Kristalle werden abgetrennt, mit kaltem Wasser nachgewaschen und bei 50°C getrocknet, wodurch die Verbindung 1 in 99,9 %iger Reinheit erhalten wird.

### Beispiel 2:

Zu einer Mischung aus 550 ml Wasser und 100ml Ehanol werden bei 71°C 65 g der verunreinigten Verbindung 1 aus Beispiel 1 und 300 ml Ethylacetat gegeben. Die Mischung wird für 30 Minuten bei 70°C gerührt, wobei zwei klare Phasen entstehen. Die wässrige Phase wird abgetrennt und über einen mit Wasserdampf beheizten Seitzfilter (Beco SD30) filtriert. Durch Zusatz von 1,5 g Methansulfonsäure wird der pH-Wert auf 1,5 eingestellt. Man lässt die wässirge Phase über Nacht unter Rühren abkühlen und rührt für weitere 3 Stunden unter Eiskühlung. Die entstandenen Kristalle werden abgetrennt, mit kaltem Wasser nachgewaschen und bei 50°C getrocknet, wodurch die Verbindung 1 in 99,9 %iger Reinheit und im Vergleich zu Beispiel 1 verbesserter Ausbeute erhalten wird.

### Beispiel 3:

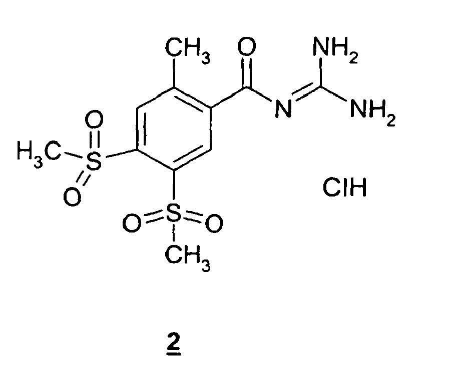

Zu 182 ml Wasser werden unter Rühren bei etwa 70°C 17,80 g der aufzureinigenden Verbindung **2** und 454ml Ethylacetat gegeben. Die Mischung wird bei 65°C für 40 Minuten gerührt, wobei zwei klare Phasen entstehen. Die wässrige Phase wird abgetrennt und mit wässriger Chlorwasserstoffsäure auf pH 1,0 eingestellt. Man lässt die wässrige Phase über Nacht unter Rühren abkühlen und rührt für weitere 3 Stunden unter Eiskühlung. Die entstandenen Kristalle werden abgetrennt, mit kaltem Wasser nachgewaschen und bei 50°C getrocknet, wodurch die Verbindung **2** in Form ihres Hydrates in 99,9 %iger Reinheit erhalten wird.

### Beispiel 4:

Zu 501 ml Wasser werden unter Rühren bei etwa 70°C 35,80 g der aufzureinigenden Verbindung **2** und 456 ml Ethylacetat gegeben. Die Mischung wird bei 65°C für 40 Minuten gerührt, wobei zwei klare Phasen entstehen. Die wässrige Phase wird abgetrennt und mit wässriger Chlorwasserstoffsäure auf pH 1,4 eingestellt. Man lässt die wässrige Phase über Nacht unter Rühren abkühlen und rührt für weitere 3 Stunden unter Eiskühlung. Die entstandenen Kristalle werden abgetrennt, mit kaltem Wasser nachgewaschen und bei 50°C getrocknet, wodurch die Verbindung 2 in Form ihres Hydrates in 99,9 %iger Reinheit erhalten wird.

### Beispiel 5:

Zu einer Mischung aus 282 ml Wasser und 51,7 g Ethanol werden unter Rühren bei etwa 70°C 40,00 g der aufzureinigenden Verbindung **2** und 113,2 g Ethylacetat gegeben. Die Mischung wird bei etwa 65°C für 10 Minuten gerührt, wobei zwei Phasen entstehen. Anschließend werden im Verlaufe von 15 Minuten weitere 30,00 g der aufzureinigenden Verbindung 2 unter Rühren eingetragen. Die wässrige Phase wird abgetrennt und mit wässriger Chlorwasserstoffsäure auf pH 1,2 eingestellt. Man lässt die wässrige Phase abkühlen und rührt für weitere 1 Stunde unter Eiskühlung. Die entstandenen Kristalle werden abgetrennt, mit kaltem Wasser nachgewaschen und bei 50°C getrocknet, wodurch die Verbindung 2 in Form ihres Hydrates in 99,9 %iger Reinheit erhalten wird.

## Patentansprüche

1. Verfahren zur Kristallisation der Verbindungen der Formel I oder deren Säureadditions-Salze, 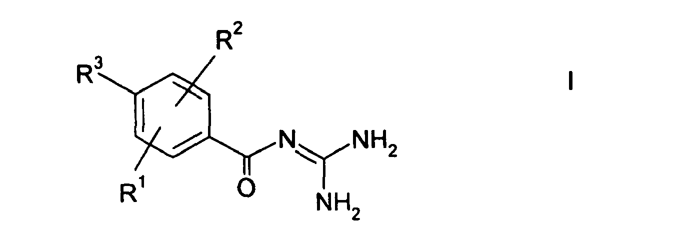 worin
R¹ R⁴SO₂- oder A,
R² und R³ unabhängig voneinander H, Hal, Alkyl mit 1 bis 12 C-Atomen, R⁴SO₂-, Ar oder Het,
R⁴ Aryl oder Alkyl mit 1 bis 12 C-Atomen,
Het einen unsubstituierten oder einfach oder mehrfach durch A, COAr, COHet und/oder Hal substituierten, gesättigten, ungesättigten oder aromatischen, mono-oder bicyclischen, heterocyclischen oder linearen oder verzweigten, ein oder mehrere Heteroatome enthaltenden organischen Rest,
Ar einen unsubstituierten oder einfach oder mehrfach durch A und/oder Hal, OH, OA, COOH, COOA, CONH₂, CONA₂, CONHA, CN, NO₂, NH₂, NHA, NA₂, NHCOA, CF₃ oder SO₂A substituierten Phenylrest,
A geradkettiges oder verzweigtes Alkyl oder Hydroxyalkyl mit 1 bis 10 C-Atomen, Alkenyl oder Alkoxyalkyl mit 2 bis 10 C-Atomen,
und
Hal F, Cl, Br, I
**dadurch gekennzeichnet, dass** man die jeweiligen Verbindungen der Formel I oder deren Mischungen mit Verunreinigungen bei gegebener Temperatur in Wasser, das mit mindestens einem mit Wasser nicht mischbaren Lösungsmittel annähernd gesättigt ist und gegebenenfalls ein oder mehrere mit Wasser mischbare Lösungsmittel enthält, löst und bei niedrigerer Temperatur die Verbindungen der Formel I kristallisieren lässt,
wobei als mit Wasser nicht mischbare Lösungsmittel Alkansäurealkylester oder aromatische Lösungsmittel verwendet werden.

2. Verfahren nach Anspruch 1 zur Kristallisation der Verbindungen der Formel Ia bis Ie 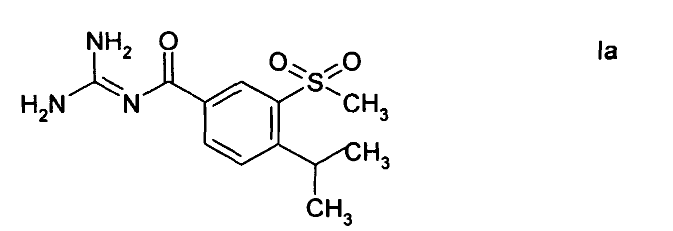 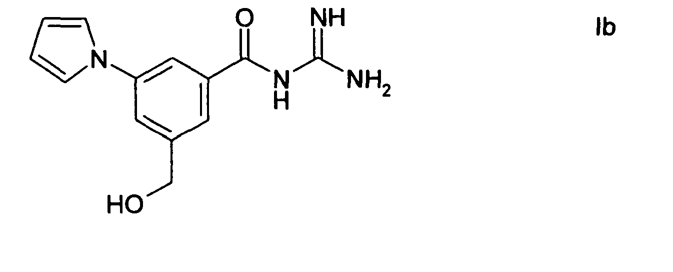 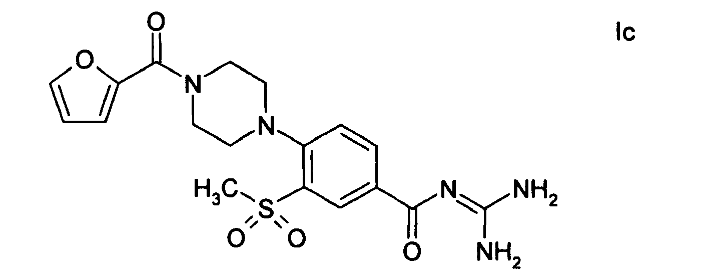 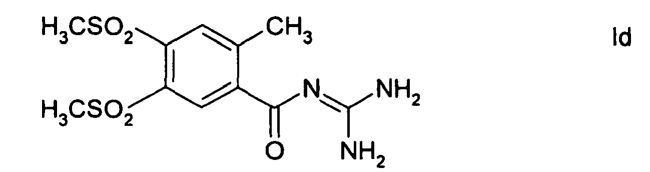 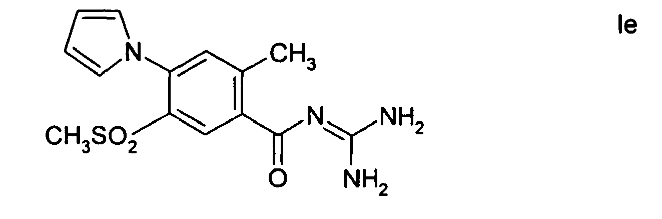 oder deren Säure-Additionssalze.

3. Verfahren nach Anspruch 1, **dadurch gekennzeichnet, dass** ein Überschuss an nicht mit Wasser mischbarem Lösungsmittel eingesetzt wird.

4. Verfahren nach einem oder mehreren der vorhergehenden Ansprüche, **dadurch gekennzeichnet, dass** ein Gemisch aus Wasser, einem oder mehreren nicht mit Wasser mischbaren Lösungsmitteln und einen oder mehreren Alkoholen verwendet wird.

5. Verfahren nach Anspruch 4, **dadurch gekennzeichnet, dass** als Alkohol Ethanol, Methanol oder n- oder iso-Propanol verwendet wird.

6. Verfahren nach Anspruch 4, **dadurch gekennzeichnet, dass** der Alkohol dem Lösungmittelgemisch in Mengen zugesetzt wird, in dem er noch keine völlige Mischbarkeit von Wasser und nicht mit Wasser mischbarem Lösungmittel vermittelt.

7. Verfahren nach Anspruch 1, **dadurch gekennzeichnet, dass** vor oder während der Kristallisation der pH-Wert mit Hilfe einer geeigneten Säure auf 1 bis 3,5 eingestellt wird.

## Claims

1. Process for the crystallisation of the compounds of the formula I or acid-addition salts thereof, 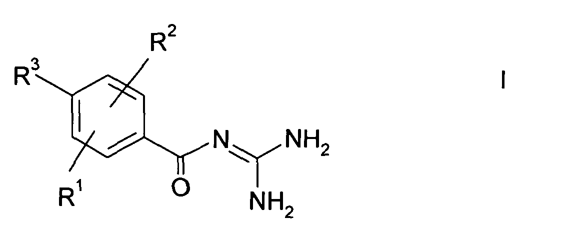 in which
R¹ denotes R⁴SO₂- or A,
R² and R³, independently of one another, denote H, Hal, alkyl having 1 to 12 C atoms, R¹SO₂-, Ar or Het,
R⁴ denotes aryl or alkyl having 1 to 12 C atoms,
Het denotes a saturated, unsaturated or aromatic, mono- or bicyclic, heterocyclic or linear or branched organic radical containing one or more heteroatoms which is unsubstituted or mono- or polysubstituted by A, COAr, COHet and/or Hal,
Ar denotes a phenyl radical which is unsubstituted or mono- or polysubstituted by A and/or Hal, OH, OA, COOH, COOA, CONH₂, CONA₂, CONHA, CN, NO₂, NH₂, NHA, NA₂, NHCOA, CF₃ or SO₂A,
A denotes straight-chain or branched alkyl or hydroxyalkyl having 1 to 10 C atoms, alkenyl or alkoxyalkyl having 2 to 10 C atoms,
and
Hal denotes F, Cl, Br, l
**characterised in that** the respective compounds of the formula I or mixtures thereof with impurities are dissolved at a given temperature in water which is virtually saturated with at least one water-immiscible solvent and optionally comprises one or more water-miscible solvents, and the compounds of the formula I are allowed to crystallise at a lower temperature,
where the water-immiscible solvents used are alkyl alkanoates or aromatic solvents.

2. Process according to Claim 1 for the crystallisation of the compounds of the formulae la to le 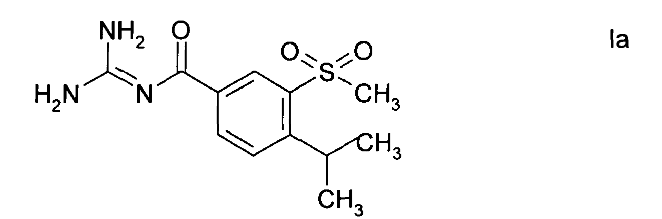 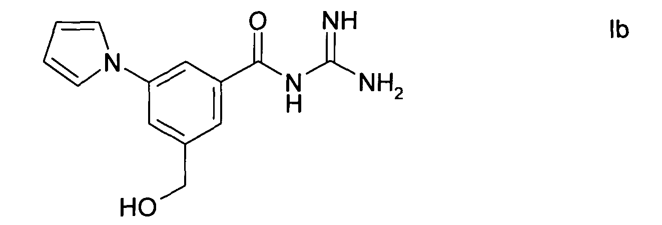 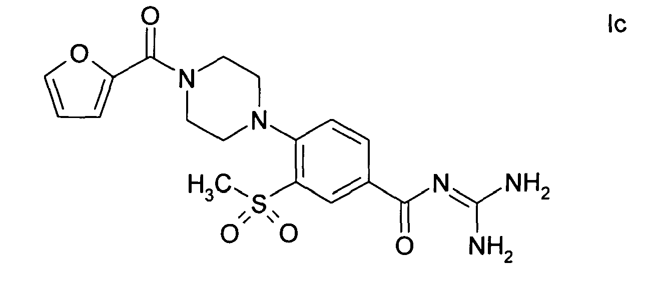 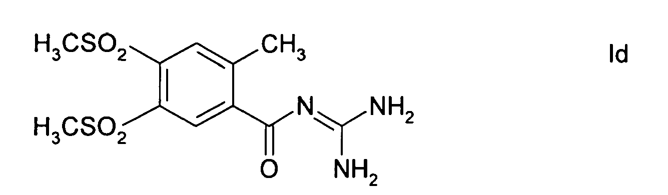 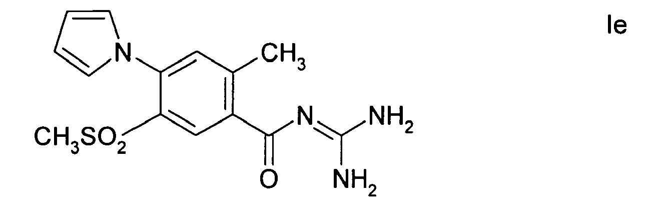 or acid-addition salts thereof.

3. Process according to Claim 1, **characterised in that** an excess of water-immiscible solvent is employed.

4. Process according to one or more of the preceding claims, **characterised in that** a mixture of water, one or more water-immiscible solvents and one or more alcohols is used.

5. Process according to Claim 4, **characterised in that** the alcohol used is ethanol, methanol or n- or isopropanol.

6. Process according to Claim 4, **characterised in that** the alcohol is added to the solvent mixture in amounts in which it does not promote complete miscibility of water and water-immiscible solvent.

7. Process according to Claim 1, **characterised in that** the pH is adjusted to 1 to 3.5 with the aid of a suitable acid before or during the crystallisation.

## Revendications

1. Procédé pour la cristallisation des composés de la formule I ou de leurs sels par addition d'acide, 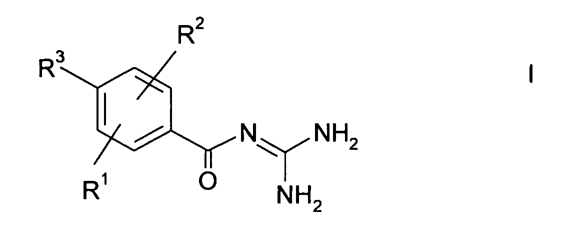 dans laquelle
R¹ représente R⁴SO₂- ou A,
R² et R³, indépendamment l'un de l'autre, représentent H, Hal, alkyle ayant 1 à 12 atomes de C, R¹SO₂-, Ar ou Het,
R⁴ représente aryle ou alkyle ayant 1 à 12 atomes de C,
Het représente un radical organique hétérocyclique ou linéaire ou ramifié, mono- ou bicyclique, saturé, insaturé ou aromatique, contenant un ou plusieurs hétéroatomes qui est non substitué ou mono- ou polysubstitué par A, COAr, COHet et/ou Hal,
Ar représente un radical phényle qui est non substitué ou mono- ou polysubstitué par A et/ou Hal, OH, OA, COOH, COOA, CONH₂, CONA₂, CONHA, CN, NO₂, NH₂, NHA, NA₂, NHCOA, CF₃ ou SO₂A,
A représente alkyle ou hydroxyalkyle en chaîne droite ou ramifié ayant 1 à 10 atomes de C, alkényle ou alcoxyalkyle ayant 2 à 10 atomes de C, et
Hal représente F, CI, Br, I
**caractérisé en ce que** les composés respectifs de la formule I ou leurs mélanges avec des impuretés sont dissous à une température donnée dans de l'eau qui est virtuellement saturée avec au moins un solvant non miscible dans l'eau et optionnellement qui comprend un ou plusieurs solvants miscibles dans l'eau, et les composés de la formule I sont amenés à cristalliser à une température basse,
dans lequel les solvants non miscibles dans l'eau utilisés sont des alcanoates d'alkyle ou des solvants aromatiques.

2. Procédé selon la revendication 1 pour la cristallisation des composés des formules la à le 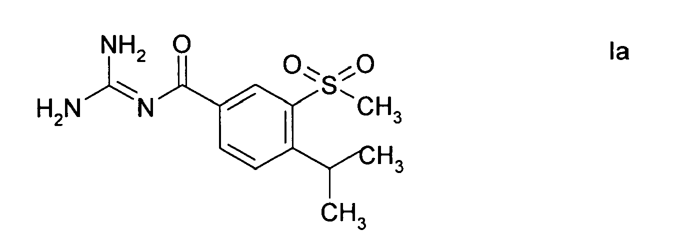 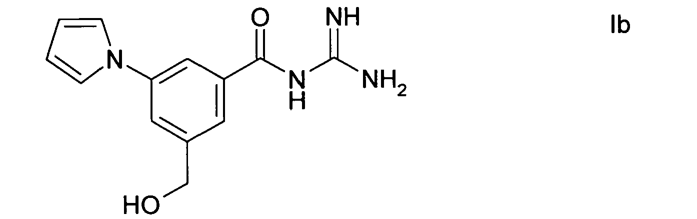 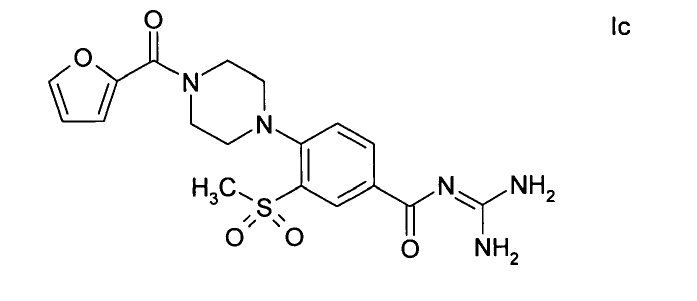 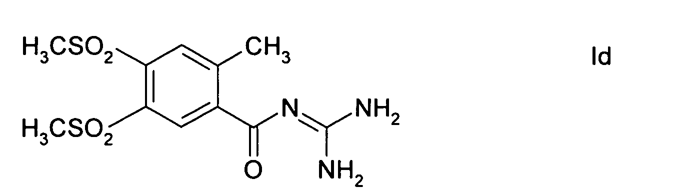 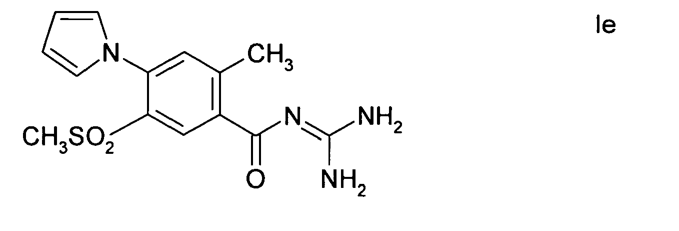 ou de leurs sels par addition d'acide.

3. Procédé selon la revendication 1, **caractérisé en ce qu'**un excès de solvant non miscible dans l'eau est employé.

4. Procédé selon une ou plusieurs des revendications précédentes, **caractérisé en ce qu'**un mélange d'eau, d'un ou plusieurs solvants non miscibles dans l'eau et d'un ou plusieurs alcools est utilisé.

5. Procédé selon la revendication 4, **caractérisé en ce que** l'alcool utilisé est éthanol, méthanol ou n- ou isopropanol.

6. Procédé selon la revendication 4, **caractérisé en ce que** l'alcool est ajouté au mélange de solvants selon des quantités selon lesquelles il ne provoque pas une complète miscibilité de l'eau et du solvant non miscible dans l'eau.

7. Procédé selon la revendication 1, **caractérisé en ce que** le pH est ajusté à 1 à 3.5 à l'aide d'un acide approprié avant ou pendant la cristallisation.
